# EUROPÄISCHE PATENTANMELDUNG

(11) **EP 4 574 009 A1**
(43) Veröffentlichungstag der Anmeldung: **25.06.2025**
(21) Anmeldenummer: 24219070.0
(22) Anmeldetag: 11.12.2024
(51) Int. Cl.: A61B 1/00

(54) **OPTISCHES TARGET, OPTISCHES KALIBRIERSYSTEM UND MEDIZINISCHES SYSTEM**

(30) Priorität: 19.12.2023 DE 102023135832
(71) Anmelder: Karl Storz SE & Co. KG, 78532 Tuttlingen (DE)
(72) Erfinder: Baumann, Christin, 78532 Tuttlingen (DE)

(57) **Zusammenfassung**

Die Erfindung betrifft ein optisches Target (20, 20', 20", 20̋‴) zur Kalibrierung einer medizinischen Bildgebungsvorrichtung (30), umfassend: einen Targetkörper (40, 40', 40", 40‴); und zumindest eine Konversionsstruktur (50, 50', 50", 50‴, 52', 52", 54', 54"), die in und/oder an dem Targetkörper (40, 40', 40", 40‴) ausgebildet ist und die dazu eingerichtet ist, Licht mit einer ersten Wellenlänge zumindest teilweise in Licht mit einer von der ersten Wellenlänge verschiedenen zweiten Wellenlänge zu wandeln; wobei zumindest die Konversionsstruktur (50, 50', 50", 50‴, 52', 52", 54', 54") generativ gefertigt ist.

Die Erfindung betrifft ferner ein optisches Kalibriersystem (12) und ein medizinisches System (10).

## Beschreibung

Die vorliegende Anmeldung betrifft ein optisches Target, ein optisches Kalibriersystem und ein medizinisches System.

In der medizinischen Bildgebung gehören Endoskope und Exoskope zum Stand der Technik, mittels derer Vergrößerungsdarstellungen von einem Untersuchungsgebiet erzeugt werden können. Weiter können durch die medizinische Bildgebung der Endoskope und/oder Exoskope unterschiedliche Schichten, beispielsweise Organe und/oder Blutgefäße und/oder sonstiges Gewebe, in unterschiedlichen Tiefen unter der Haut und/oder unter den Organen, in die das Endoskop eingeführt ist, visualisiert werden.

Zusätzlich können durch die medizinische Bildgebung der Endoskope unterschiedliche Gewebearten, beispielsweise Organe und/oder Blutgefäße und/oder Tumorgewebe, in unterschiedlichen Tiefen unter der Haut oder in der Körperhöhle visualisiert werden. Um die Gewebearten besser differenzieren zu können, werden Fluoreszenzbildgebungsverfahren eingesetzt. Dazu wird dem Patienten ein Medikament mit einem Fluoreszenzfarbstoff, insbesondere Fluorophoren, verabreicht, das sich in einer der unterschiedlichen Gewebearten ablagert. Weiter können auch multimodale medizinische Bildgebungsverfahren eingesetzt werden.

Zusammenfassend, erhalten Chirurgen durch die medizinische Bildgebung sehr genaue Informationen darüber, bis in welche Tiefe sie schneiden müssen, um beispielsweise die betreffenden Gewebe erreichen und diese entfernen zu können und können daher eigene Handlungen intuitiver über einen Bildschirm einschätzen und/oder verfolgen. Weiter können zusätzlich zu den Gewebeteilen, die entfernt werden müssen, Gewebe visualisiert werden, die beispielsweise auf keinen Fall verletzt werden dürfen.

Zum Testen bzw. Evaluieren, ob die Endoskope und/oder Exoskope die Fluoreszenzen in ausreichender Eindringtiefe und/oder in einem ausreichenden Abstand zum Endoskop und/oder Exoskop richtig identifizieren können, wird in bekannten Testaufbauten weiter ein Silikonschlauch in das streuende Medium eingebracht, der mit einer bestimmten Konzentration von fluoreszierendem Farbstoff gefüllt ist. Problematisch ist jedoch, dass die Konzentration des fluoreszierenden Farbstoffs über die Zeit durch Fotobleaching abnimmt. Das bedeutet, dass die Fluoreszenzeigenschaft des Silikonschlauchs in Abhängigkeit der Lagerzeit stark variiert. Der obige Testaufbau ist weiter derart empfindlich, dass bereits kleine Fehler beim Anmischen des Fluoreszenzfarbstoffs für den Silikonschlauch und schon eine anschließende Lagerung am Tag des Experiments aufgrund des Fotobleachings zu höchst ungenauen Testbedingungen führen.

Weiter ist es durch die obige Anordnung nicht möglich, komplexere Testaufbauten zu simulieren. Komplexere Testaufbauten werden aber zum Evaluieren multimodaler medizinischer Bildgebungsverfahren dringend benötigt, um testen zu können, ob unterschiedliche Fluoreszenzen in unterschiedlichen Eindringtiefen von dem medizinischen Bildgebungsverfahren realitätsnah wiedergegeben werden können.

Ausgehend vom Stand der Technik liegt der Erfindung die Aufgabe zugrunde, eine Bildgebungsvorrichtung einfach zu überprüfen.

Diese Aufgabe wird erfindungsgemäß durch ein optisches Target, ein optisches Kalibriersystem und ein medizinisches System gelöst, wie sie hierin beschrieben und in den Ansprüchen definiert sind.

Die vorliegende Erfindung sieht dazu ein optisches Target zur Kalibrierung einer medizinischen Bildgebungsvorrichtung vor. Das optische Target umfasst einen Targetkörper; und zumindest eine Konversionsstruktur, die in und/oder an dem Targetkörper ausgebildet ist. Ferner ist die Konversionsstruktur dazu eingerichtet, Licht mit einer ersten Wellenlänge zumindest teilweise in Licht mit einer von der ersten Wellenlänge verschiedenen zweiten Wellenlänge zu wandeln. Weiter ist zumindest die Konversionsstruktur generativ gefertigt.

Das optische Target kann zur stereoskopischen Kalibrierung der medizinischen Bildgebungsvorrichtung und/oder zur Kalibrierung der Fluoreszenzbildgebungsverfahren der medizinischen Bildgebungsvorrichtung eingesetzt werden. Insbesondere kann durch das optische Target die Stereonullebene der stereoskopischen Bildgebung der medizinischen Bildgebungsvorrichtung kalibriert werden.

Die medizinische Bildgebungsvorrichtung kann insbesondere ein Exoskop sein. Alternativ oder zusätzlich kann die Bildgebungsvorrichtung beispielsweise ein Endoskop sein. Die medizinische Bildgebungsvorrichtung kann dazu eingerichtet sein, Bilder eines Untersuchungsgebiets aufzunehmen. In einigen Ausführungsformen kann die Bildgebungsvorrichtung dazu eingerichtet sein, Vergrößerungsdarstellungen des Untersuchungsgebiets zu erzeugen. Die Darstellung kann während des Durchführens eines medizinischen Eingriffs und/oder während einer diagnostischen Aktion beobachtet werden. Die medizinische Bildgebungsvorrichtung kann mobil und/oder bewegbar sein. Es kann vorgesehen sein, dass die Bildgebungsvorrichtung beispielsweise in einem OP-Saal bewegbar ist. Benötigt ein Benutzer beispielsweise Unterstützung durch Bildgebung, kann die Bildgebungsvorrichtung zu dem Einsatzort, also etwa einem Patienten, bewegbar sein. Die Bildgebungsvorrichtung kann auch in sich selbst beweglich sein. Die Bildgebungsvorrichtung kann beispielsweise einen beweglichen Haltearm umfassen und/oder die Bildgebungsvorrichtung an dem beweglichen Haltearm angeordnet sein.

Der Targetkörper definiert insbesondere zumindest eine Außenfläche des optischen Targets. Zumindest aus einer Raumrichtung kann Licht durch den Targetkörper in das optische Target eingekoppelt bzw. eingeleitet werden. Vorzugsweise kann aus allen Raumrichtungen Licht durch den Targetkörper in das optische Target eingekoppelt bzw. eingeleitet werden. Beispielsweise wird zumindest durch eine Außenfläche des Targetkörpers, vorzugsweise aus allen Außenflächen des Targetkörpers, Licht in das optische Target eingekoppelt bzw. eingeleitet.

Generative Fertigung bzw. "additive manufacturing" beschreibt insbesondere ein Verfahren, mit dem ein Objekt schichtweise aufgebaut wird, beispielsweise ein 3D-Druckverfahren. Ausgangsmaterialien zur generativen Fertigung sind insbesondere Formsande, Polymergipse, Acrylharze, Kunststoffe, Metalle und/oder Acrylglas. Vorzugsweise wird in der vorliegenden Erfindung zur generativen Fertigung Acrylglas verwendet.

Ferner kann die Konversionsstruktur für Licht mit der ersten Wellenlänge teildurchlässig sein. Beispielsweise kann die Konversionsstruktur dazu eingerichtet sein 10 % - 100 %, insbesondere 30 % - 80 %, vorzugsweise 40 % - 60 %, der Lichtmenge und/oder des Lichtstroms des Lichts mit der ersten Wellenlänge in Licht mit der zweiten Wellenlänge zu wandeln. Außerdem kann die Konversionsstruktur dazu eingerichtet sein, entsprechend der übrigen Merkmale Licht in engen Spektralbändern zu wandeln. Eng kann bedeuten, dass das gewandelte Spektralband beispielsweise bis zu 200 nm, insbesondere 100 nm, vorzugsweise 50 nm, besonders bevorzugt 20 nm breit ist. Die Konversionsstruktur kann insbesondere dazu eingerichtet sein, Licht mit der zweiten Wellenlänge abzustrahlen. Das abgestrahlte Licht kann zur Ausleuchtung des optischen Targets verwendet werden. Alternativ oder zusätzlich kann Licht mit der ersten Wellenlänge zur Ausleuchtung des optischen Targets verwendet werden. Beispielsweise kann die Konversionsstruktur dazu eingerichtet sein, zeitlich abwechselnd Licht zu wandeln und durchzulassen.

Die Konversionsstruktur kann zur Lichtkonversion eingerichtete Filter umfassen. Die Konversionsstruktur kann insbesondere dazu eingerichtet sein, Licht mit der ersten Wellenlänge zu absorbieren und Licht mit der zweiten Wellenlänge zu emittieren. Ferner kann in einigen Ausführungsformen alternativ oder zusätzlich mittels der Konversion die Wellenlänge des Lichts mit der ersten Wellenlänge halbierbar sein, beispielsweise durch Frequenzverdopplung (second harmonic generation, SHG) und/oder Frequenzvervielfachung mittels nicht linearer optischer Prozesse. Beispielsweise kann die zweite Wellenlänge eine Wellenlänge aus dem ultravioletten Bereich sein. Ferner können zur Lichtkonversion Quantenpunkte vorgesehen sein, beziehungsweise der Konversionsbereich Quantenpunkte umfassen.

Durch die erfindungsgemäße Ausgestaltung kann Tageslicht und/oder eine externe Lichtquelle, die ebenfalls zur Beleuchtung des Raums vorgesehen ist, bereits ausreichen, damit das optische Target ausreichend zur Kalibrierung der medizinischen Bildgebungsvorrichtung beleuchtet ist.

Insbesondere kann durch die erfindungsgemäßen Merkmale ein optisches Target bereitgestellt werden, das über einen erhöhten Zeitraum vergleichbare Eigenschaften aufweist, benutzerfreundlich handhabbar ist und nicht durch Benutzerfehler negativ beeinflusst werden kann.

Zur einfachen und kostengünstigen Herstellung kann der Targetkörper um die Konversionsstruktur gegossen sein.

Weiter kann der Targetkörper generativ zusammen mit der Konversionsstruktur gefertigt sein. Dazu kann der Targetkörper beispielsweise zeitgleich mit der Konversionsstruktur gedruckt werden, indem während des 3D-Druckverfahrens geeignet zwischen zwei Materialien umgeschaltet wird

Weiter kann der Targetkörper aus Acrylgas gebildet sein, damit einfach Licht in das optische Target eingeleitet werden kann.

Beispielsweise weist die wenigstens eine Konversionsstruktur eine rechteckige und/oder runde Form und/oder die Form eines anatomischen Gefäßes wie beispielsweise eines Blutgefäßes auf. Die Form des Konversionsbereichs kann einem Blutgefäß nachempfunden sein. Beispielsweise kann der Konversionsbereich zylindrisch und/oder röhrenförmig sein. Dadurch können bei der Kalibrierung der medizinischen Bildgebungsvorrichtung optische Targets eingesetzt werden, die realitätsnahe Strukturen nachstellen bzw. ggf. vereinfachte, aber realistische Strukturen nachstellen, die beispielsweise bei einer Operation durch das optische Bildgebungssystem aufgezeichnet werden.

Zur Bildung von komplexeren realitätsnahen Strukturen, kann das optische Target weiter wenigstens zwei Konversionsstrukturen aufweisen. Weiter können die ersten und/oder zweiten Wellenlängen der zwei Konversionsstrukturen unterschiedlich sein. Beispielsweise kann eine erste Konversionsstruktur dazu eingerichtet sein, Licht mit einer ersten Wellenlänge zumindest teilweise in zweites Licht mit einer von der ersten Wellenlänge verschiedenen zweiten Wellenlänge zu wandeln. Ferner kann eine zweite Konversionsstruktur dazu eingerichtet sein, Licht mit einer dritten Wellenlänge zumindest teilweise in zweites Licht mit einer von der dritten Wellenlänge verschiedenen vierten Wellenlänge zu wandeln. Hierbei kann sich die erste Wellenlänge von der dritten Wellenlänge unterscheiden. Alternativ oder zusätzlich kann sich die zweite Wellenlänge von der vierten Wellenlänge unterscheiden.

Um sicherzustellen, dass die medizinische Bildgebungsvorrichtung die gewünschte Eindringtiefe, beispielsweise für die Operation notwendige Eindringtiefe aufweist, kann das optische Target mehrere Konversionsstrukturen aufweisen, die übereinander und/oder nebeneinander angeordnet sind.

Um eine noch realitätsnähere Kalibrierung der medizinischen Bildgebungsvorrichtung zu realisieren, kann durch wenigstens zwei Konversionsstrukturen ein Blutgefäß und/oder Organ und/oder ein Körperteil zumindest teilweise nachgebildet sein. Weiter kann das optische Target, um ein Organ zu simulieren und/oder um unterschiedliche Marker/ Fluoreszenzlösungen nachzustellen, die in unterschiedlichen Teilen/Schichten der Organe oder Gefäße der Organe gelöst werden, mehrere Konversionsstrukturen mit unterschiedlichen Umwandlungseigenschaften von Wellenlängen aufweisen.

In einer besonders einfachen und kostengünstigen Ausgestaltung des optischen Targets kann die Konversionsstruktur den Targetkörper zumindest im Wesentlichen vollständig und insbesondere komplett ausfüllen. Der Targetkörper kann, insbesondere vollständig, aus einem Material gefertigt sein, das dazu eingerichtet ist, Licht mit einer ersten Wellenlänge zumindest teilweise in zweites Licht mit einer von der ersten Wellenlänge verschiedenen zweiten Wellenlänge zu wandeln.

Damit das optische Target besser von der medizinischen Bildgebungsvorrichtung identifiziert werden kann, insbesondere ohne Verwendung von zusätzlichen externen Lichtquellen, und/oder zur Bildung einer realitätsnahen Kalibrierumgebung, insbesondere zur Nachbildung der Streuung von Haut, kann die Oberfläche des Targetkörpers insbesondere Licht streuen.

Zur kostengünstigen Herstellung kann insbesondere die Oberfläche des Targetkörpers aufgeraut und/oder sandgestrahlt sein.

Damit das optische Target besser für die medizinische Bildgebungsvorrichtung sichtbar ist, insbesondere ohne Verwendung von zusätzlichen externen Lichtquellen, und/oder zur Bildung einer realitätsnahen Kalibrierumgebung, kann das optische Target weiter insbesondere ein Streuungselement aufweisen. Der Targetkörper kann hierfür in das Streuungselement eingebettet sein. Alternativ kann das Streuungselement ein Teil des Targetkörpers sein, insbesondere in diesen aufgenommen sein. Das Streuungselement kann die Konversionsstruktur oder die Konversionsstrukturen ganz oder teilweise umgeben.

Insbesondere bildet das Streuungselement und/oder der Targetkörper, zur Bildung einer realitätsnahen Kalibrierumgebung, die Streuung von menschlichem Gewebe nach.

Des Weiteren kann ein optisches Kalibriersystem vorgesehen sein. Das optische Kalibriersystem umfasst ein erfindungsgemäßes Target und eine Halterung, die dazu vorgesehen ist, das optische Target an die zu prüfende, beispielsweise stereoskopische medizinische Bildgebungsvorrichtung ankoppelbar zu machen. Mittels der Halterung kann erreicht werden, dass das Überprüfen einer insbesondere optischen Kalibrierung reproduzierbar durchführbar ist. Ferner ist das Kalibriersystem durch die Halterung einfach bedienbar, insbesondere indem das Kalibriersystem effizient und einfach bezüglich der Bildgebungsvorrichtung ausrichtbar ist.

Die Halterung kann zudem einen Abstandshalter umfassen, der einen Abstand zwischen der zu prüfenden medizinischen Bildgebungsvorrichtung und dem Target definiert. Der Abstand kann beispielsweise in etwa dem Abstand entsprechen, den die Bildgebungsvorrichtung, insbesondere eine Bilderfassungseinheit und/oder Eingangsoptik der Bildgebungsvorrichtung, üblicherweise in einem Betrieb zu einem zu untersuchenden Objekt haben kann. Außerdem kann der Abstand speziell mit dem optischen Target und/oder mit der Bildgebungsvorrichtung abstimmbar sein. Der Abstand kann zumindest im Wesentlichen einer Brennweite einer ersten Bilderfassungseinrichtung und/oder einer Brennweite einer zweiten Bilderfassungseinrichtung der medizinischen Bildgebungsvorrichtung entsprechen. In einigen Ausführungsformen kann der Abstand wenigstens 5 cm, wenigstens 10 cm oder wenigstens 20 cm und/oder höchstens 100 cm, höchstens 80 cm oder höchstens 50 cm betragen. Eine Reproduzierbarkeit der Überprüfung kann erhöht werden. Ferner kann die Überprüfung standardisierbar sein.

Zudem kann die Halterung dazu eingerichtet sein, in einem an die zu kalibrierende medizinische Bildgebungsvorrichtung angekoppelten Zustand das Eigengewicht des Kalibriersystems zu tragen. Das kann bedeuten, dass das Kalibriersystem derart an die Bildgebungsvorrichtung ankoppelbar sein kann, dass das Kalibriersystem nicht auf dem Boden steht. Das heißt, das Kalibriersystem muss kein Standbein und/oder dergleichen umfassen. Das System aus Kalibriersystem und Bildgebungsvorrichtung kann dadurch insbesondere dynamisch besser koppelbar sein. Vorteilhafterweise können weniger Schwingungen und/oder eine geringere Schwingungsenergie über den Boden auf das Kalibriersystem übertragbar sein. Eine Genauigkeit der Überprüfung kann verbessert werden. Ferner kann das Kalibriersytem kompakter ausgebildet sein.

Die Halterung kann ferner Öffnungen aufweisen, durch die Umgebungslicht auf den Targetkörper gelangen kann bzw. um zu gewährleisten, dass das Umgebungslicht auf den Targetkörper fällt. Beispielsweise können an der Halterung weiter Spiegel angebracht sein, um einen Anteil des Umgebungslichts zu erhöhen, der auf den Targetkörper fällt.

Die Halterung kann außerdem einen Koppelabschnitt zur Kopplung mit der zu prüfenden medizinischen Bildgebungsvorrichtung umfassen, wobei der Koppelabschnitt einen Vorsprung umfasst, der dazu eingerichtet ist, das Kalibriersystem durch ein Hintergreifen zu halten. Die Bildgebungsvorrichtung kann zudem einen Halteabschnitt umfassen, an den die Halterung koppelbar ist. Beispielsweise kann der Vorsprung den Halteabschnitt zumindest teilweise hintergreifen und dazu eingerichtet sein, während des Koppelns des Kalibriersystems dieses durch ein Hintergreifen, insbesondere zumindest abschnittsweise des Halteabschnitts, zu halten. Das Kalibriersystem kann etwa während des Koppelns um den Vorsprung gedreht werden, insbesondere, während dieser den Halteabschnitt teilweise hintergreift. Der Vorsprung kann in dem Halteabschnitt drehbar gelagert sein. Das Kalibriersystem, insbesondere der Koppelabschnitt und/oder der Vorsprung, und die Bildgebungsvorrichtung, insbesondere der Halteabschnitt, können gemeinsam eine Verbindung ausbilden. Die Verbindung kann beispielsweise eine Schwalbenschwanzverbindung umfassen. Dadurch kann ein kompaktes und/oder effizientes Kalibriersystem vorsehbar sein.

Der Koppelabschnitt kann ferner ein bewegbares Halteelement umfassen, das dazu eingerichtet ist, die Halterung wahlweise an der zu prüfenden medizinische Bildgebungsvorrichtung zu fixieren oder von dieser zu lösen. Beispielsweise kann mittels des Halteelements die Halterung an die Bildgebungsvorrichtung klemmbar sein. Alternativ oder zusätzlich kann das bewegbare Halteelement dazu eingerichtet sein, die Bildgebungsvorrichtung, insbesondere den Halteabschnitt, abschnittsweise zu hintergreifen. Das bewegbare Halteelement kann etwa ein spannbares Halteelement, insbesondere eine Klemmfeder, eine Stellfeder und/oder dergleichen, eine Schraube und/oder eine Rastnase umfassen. Dadurch kann das Kalibriersystem in einfacher Weise an die Bildgebungsvorrichtung wahlweise fixierbar und/oder von dieser lösbar sein. Ein Ankoppeln kann in kurzer Zeit und/oder flexibel erfolgen.

Weiter umfasst die Erfindung ein medizinisches System mit einer medizinische Bildgebungsvorrichtung und einem optischen Target zur Überprüfung einer stereoskopischen Kalibrierung der medizinische Bildgebungsvorrichtung. Weiter kann die zu prüfende medizinischen Bildgebungsvorrichtung einen Fluoreszenzkanal und/oder einen Weißlichtkanal aufweisen.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Figur 1: eine beispielhafte Ausführungsform eines erfindungsgemäßen optischen Targets;
- Figur 2: eine weitere beispielhafte Ausführungsform eines erfindungsgemäßen optischen Targets;
- Figur 3: eine weitere beispielhafte Ausführungsform eines erfindungsgemäßen optischen Targets;
- Figur 4: eine beispielhafte Ausführungsform eines erfindungsgemäßen medizinischen Systems mit einer medizinische Bildgebungsvorrichtung und einem erfindungsgemäßen optischen Target zur Überprüfung einer stereoskopischen Kalibrierung der medizinischen Bildgebungsvorrichtung.

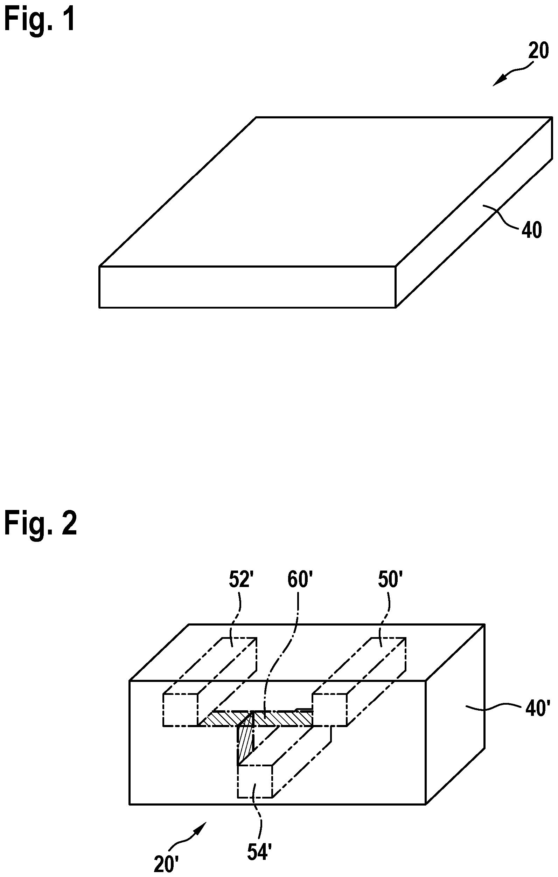

Figur 1 zeigt eine beispielhafte Ausführungsform eines optischen Targets 20 mit einem Targetkörper 40. Der Targetkörper 40 kann beispielsweise aus Glas und/oder Acrylglas gebildet sein. Beispielsweise wird der Targetkörper 40 in einem generativen Fertigungsverfahren, insbesondere einem 3D-Druckverfahren, gefertigt. Der gesamte Targetkörper 40 kann eine Konversionsstruktur bilden.

Figur 2 zeigt eine weitere beispielhafte Ausführungsform eines weiteren optischen Targets 20' mit einem Targetkörper 40'. Wie dargestellt, können in den Targetkörper 40' mehrere Konversionsstrukturen 50', 52' und/oder 54" aufgenommen sein. Beispielsweise können, wie dargestellt, die Konversionsstrukturen 50' und 52" nebeneinander angeordnet sein und die Konversionsstruktur 54' kann unter und ggf. zwischen den Konversionsstrukturen 50' und 52" angeordnet sein. Es sind aber viele Formen, Anordnungen und/oder Anzahlen von Konversionsstrukturen möglich, insbesondere um eine realitätsnahe Kalibrierumgebung für die medizinische Bildgebungsvorrichtung zu gewährleisten. Die Konversionsstrukturen 50', 52' und/oder 54" können durch ein Verbindungselement 60' miteinander verbunden sein. Insbesondere können die Konversionsstrukturen 50', 52' und/oder 54" und die Verbindungselemente 60' durch ein generatives Fertigungsverfahren, insbesondere ein 3D-Druckverfahren, gefertigt sein. Darauffolgend kann beispielsweise der Targetkörper 40' um die Konversionsstrukturen 50', 52' und/oder 54" und/oder das Verbindungselement 60' gegossen werden.

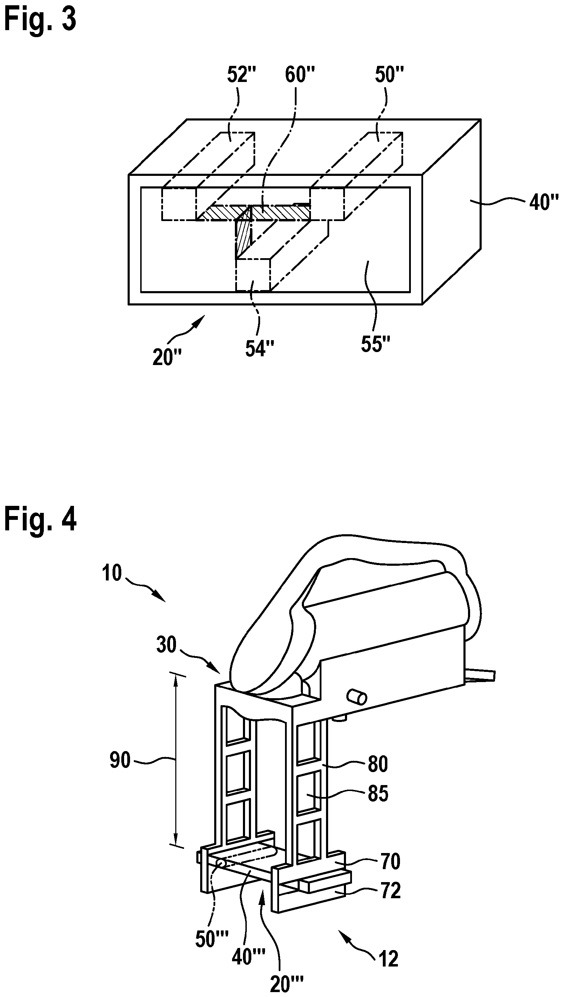

Figur 3 zeigt eine weitere beispielhafte Ausführungsform eines weiteren optischen Targets 20", mit einem Targetkörper 40", Konversionsstrukturen 50", 52" und 54" und einem Verbindungselement 60". Prinzipiell sind Konversionsstrukturen 50", 52" und 54" und das Verbindungselement 60" vergleichbar zu der beispielhaften Ausführungsform angeordnet, die in Figur 2 dargestellt ist. Es sind aber viele Formen, Anordnungen und/oder Anzahlen von Konversionsstrukturen möglich, insbesondere um eine realitätsnahe Kalibrierumgebung für die medizinische Bildgebungsvorrichtung zu gewährleisten. Der Targetkörper 40" kann beispielsweise ein vorgefertigter Körper sein, der hohl ausgebildet und beispielsweise an zumindest einer Seite geöffnet ist. Der Targetkörper 40" kann beispielsweise aus Glas und/oder Acrylglas gefertigt sein. Die Konversionsstrukturen 50", 52" und/oder 54" und/oder das Verbindungselement 60" sind in den Targetkörper 40" aufgenommen. Weiter können die Konversionsstrukturen 50", 52" und/oder 54" und/oder das Verbindungselement 60"innerhalb des Targetkörpers 40" von einem Streuungselement 55" umgeben sein. Das Streuungselement 55" kann beispielsweise um die Konversionsstrukturen 50", 52" und/oder 54" und/oder das Verbindungselement 60" herum gegossen werden und/oder zusammen mit den Konversionsstrukturen 50", 52" und/oder 54" und/oder dem Verbindungselement 60" und/oder dem Targetkörper 40" generativ gefertigt werden.

Figur 4 zeigt eine schematische Darstellung eines medizinischen Systems 10 mit einer medizinischen Bildgebungsvorrichtung 30 und einem optischen Target 20‴. Das optische Target 20‴ umfasst einen Targetkörper 40‴ und eine Konversionsstruktur 50‴, die beispielhaft rund ausgestaltet ist. Das optische Target 20‴ ist Teil eines optischen Kalibriersystems 12. Weiter umfasst das optische Target 20‴ eine Halterung 70, in die der Targetkörper 40‴ eingeführt werden kann. Vorliegend weist die Halterung 70 eine Aufnahme 72 für das optische Target 20‴ auf. An der Aufnahme 72 ist ein Ende eines Abstandshalters 80 angebracht. An dem gegenüberliegenden Ende des Abstandshalters 80, das mit der Aufnahme 72 verbunden ist, ist die medizinischen Bildgebungsvorrichtung 30 aufgesteckt. Durch den Abstandshalter 80 kann zwischen dem Targetkörper 40‴ und der medizinischen Bildgebungsvorrichtung 30 ein Abstand 90 realisiert werden. Die Abstandshalterung 80 weist weiter Löcher 85 auf, damit mehr Umgebungslicht auf den Targetkörper 40‴ treffen kann bzw. damit ein Schattenwurf auf den Targetkörper 40" verhindert wird.

Die medizinische Bildgebungsvorrichtung 30 kann eine erste Bilderfassungseinrichtung und eine zweite Bilderfassungseinrichtung umfassen, die jeweils in dem ersten Spektralbereich lichtempfindlich sind. Ferner kann die medizinische Bildgebungsvorrichtung 30 eine dritte Bilderfassungseinrichtung und vierte Bilderfassungseinrichtung umfassen, die jeweils in dem weiteren Spektralbereich lichtempfindlich sind. Mittels der ersten Bilderfassungseinrichtung und der zweiten Bilderfassungseinrichtung kann beispielsweise eine Stereobilderfassung in dem ersten Spektralbereich durchführbar werden. Mittels der dritten Bilderfassungseinrichtung und der vierten Bilderfassungseinrichtung kann beispielsweise eine Stereobilderfassung in dem zweiten Spektralbereich durchgeführt werden. Insbesondere liegt der zweite Spektralbereich in dem Nahinfrarotbereich. Dadurch kann eine Fluoreszenzstereobilderfassung mittels der dritten Bilderfassungseinrichtung und der vierten Bilderfassungseinrichtung durchführbar sein.

Um eine Fluoreszenzbildgebung zu ermöglichen, können die Bilderfassungseinrichtungen weiter Bildsensoren umfassen, die in unterschiedlichen Spektralbereichen lichtempfindlich sind. Die erste Bilderfassungseinrichtung und die zweite Bilderfassungseinrichtung können jeweils einen ersten Bildsensor umfassen, der zumindest überwiegend in dem ersten Spektralbereich, der insbesondere dem sichtbaren Licht zugeordnet ist, lichtempfindlich ist. D.h., mittels des ersten Bildsensors ist eine Bilderfassung im Wellenlängenbereich des sichtbaren Lichts durchführbar. Dies entspricht etwa der gängigen Bilderfassung. Mittels des zweiten Bildsensors ist eine Bilderfassung im Wellenlängenbereich des nahinfraroten Lichts durchführbar.

Zur Kalibrierung einer solchen medizinische Bildgebungsvorrichtung 30 kann das optische Target 20‴ durch beispielsweise mehrere, insbesondere zwei, Konversionsstrukturen Licht in beiden Spektralbereichen bereitstellen, um die geometrische Kalibrierung der medizinischen Bildgebungsvorrichtung 30 überprüfen zu können. Zur Überprüfung der geometrischen Kalibrierung nimmt die medizinische Bildgebungsvorrichtung 30 das Target 20‴ bzw. die wenigstens eine Konversionsstruktur 50‴ auf. Insbesondere nimmt jede der Bilderfassungseinrichtungen jeweils zumindest ein Bild der Targetkörpers 40‴ mit wenigstens einer Konversionsstruktur 50‴ auf. Anhand der Bilder der wenigstens einen Konversionsstruktur 50‴ des optischen Targets 20‴ kann beispielsweise der Benutzer die optische Kalibrierung der Bilderfassungseinrichtungen überprüfen.

### Bezugszeichenliste

- 10: medizinisches System
- 20, 20', 20", 20‴: optisches Target
- 30: medizinische Bildgebungsvorrichtung
- 40, 40', 40", 40‴: Targetkörper
- 50, 50', 50", 50‴: Konversionsstruktur
- 52', 52": Konversionsstruktur
- 54', 54": Konversionsstruktur
- 55: Streuungselement
- 60: Verbindungselement
- 70: Halterung
- 72: Aufnahme
- 80: Abstandshalter
- 90: Abstand

## Patentansprüche

1. Optisches Target (20, 20', 20", 20‴) zur Kalibrierung einer medizinischen Bildgebungsvorrichtung (30), umfassend:
einen Targetkörper (40, 40', 40", 40‴); und
zumindest eine Konversionsstruktur (50, 50', 50", 50‴, 52', 52", 54', 54"), die in und/oder an dem Targetkörper (40, 40', 40", 40‴) ausgebildet ist und die dazu eingerichtet ist, Licht mit einer ersten Wellenlänge zumindest teilweise in Licht mit einer von der ersten Wellenlänge verschiedenen zweiten Wellenlänge zu wandeln;
wobei zumindest die Konversionsstruktur (50, 50', 50", 50‴, 52', 52", 54', 54") generativ gefertigt ist.

2. Optisches Target nach Anspruch 1,
wobei der Targetkörper (40, 40', 40", 40‴) um die Konversionsstruktur (50, 50', 50", 50'", 52`, 52", 54`, 54") gegossen ist; und/oder
wobei der Targetkörper (40, 40', 40", 40‴) generativ zusammen mit der Konversionsstruktur (50, 50', 50", 50'", 52`, 52", 54`, 54") gefertigt ist.

3. Optisches Target (20, 20', 20", 20‴) nach Anspruch 1 oder 2,
wobei das optische Target aus Acrylgas gebildet ist.

4. Optisches Target (20, 20', 20", 20‴) nach einem der vorhergehenden Ansprüche,
wobei die wenigstens eine Konversionsstruktur (50, 50', 50", 50‴, 52', 52", 54', 54") eine rechteckige und/oder runde Form oder die Form eines anatomischen Gefäßes aufweist.

5. Optisches Target (20, 20', 20", 20‴) nach einem der vorhergehenden Ansprüche, weiter umfassend:
wenigstens zwei Konversionsstrukturen (50, 50', 50", 50‴, 52`, 52", 54`, 54"),
wobei die ersten und/oder zweiten Wellenlängen der wenigstens zwei Konversionsstrukturen (50, 50', 50", 50‴, 52', 52", 54', 54") unterschiedlich sind.

6. Optisches Target (20, 20', 20", 20‴) nach einem der vorhergehenden Ansprüche,
wobei mehrere Konversionsstrukturen (50, 50', 50", 50‴, 52', 52", 54', 54") übereinander und/oder nebeneinander angeordnet sind.

7. Optisches Target (20, 20', 20", 20‴) nach einem der vorhergehenden Ansprüche,
wobei durch mehrere Konversionsstrukturen (50, 50', 50", 50''', 52', 52", 54', 54") die Form eines anatomischen Gefäßes und/oder Organs und/oder eines Körperteils zumindest teilweise nachgebildet ist.

8. Optisches Target (20, 20', 20", 20‴) nach einem der vorhergehenden Ansprüche,
wobei die Oberfläche des Targetkörpers (40, 40', 40", 40‴) Licht streut.

9. Optisches Target (20, 20', 20", 20‴) nach einem der vorhergehenden Ansprüche,
wobei die Oberfläche des Targetkörpers (40, 40', 40", 40‴) aufgeraut und/oder sandgestrahlt ist.

10. Optisches Target (20, 20', 20", 20‴) nach einem der vorhergehenden Ansprüche,
**dadurch gekennzeichnet, dass** es ein Streuungselement (55) aufweist, wobei der Targetkörper (40, 40', 40", 40‴) in das Streuungselement (55) eingebettet ist oder das Streuungselement (55) in den Targetkörper (40, 40', 40", 40‴) aufgenommen ist.

11. Optisches Target (20, 20', 20", 20‴) nach Anspruch 10,
wobei das Streuungselement (55) und/oder der Targetkörper (40, 40', 40", 40‴) die Streuung von menschlichem Gewebe nachbildet.

12. Optisches Kalibriersystem (12), umfassend:
ein optisches Target (20, 20', 20", 20‴) nach einem der vorhergehenden Ansprüche; und
eine Halterung (70), die dazu vorgesehen ist, das optische Target (20, 20', 20", 20‴) an die zu prüfende medizinische Bildgebungsvorrichtung (30) ankoppelbar zu machen.

13. Optisches Kalibriersystem (12) nach Anspruch 12,
wobei die Halterung (70) einen Abstandshalter (80) umfasst, der einen Abstand (90) zwischen der zu prüfenden medizinischen Bildgebungsvorrichtung (30) und dem optischen Target (20, 20', 20", 20‴) definiert.

14. Medizinisches System (10), umfassend:
eine medizinische Bildgebungsvorrichtung (30);
und ein optisches Target (20, 20', 20", 20‴) zur Überprüfung einer stereoskopischen Kalibrierung der medizinischen Bildgebungsvorrichtung (30) nach einem der Ansprüche 1 bis 11 und/oder ein optisches Kalibriersystem (12) nach Anspruch 12 oder 13.

15. Medizinisches System (10) nach Anspruch 14,
wobei die zu prüfende medizinische Bildgebungsvorrichtung (30) einen Fluoreszenzkanal und/oder einen Weißlichtkanal aufweist.
